# EUROPEAN PATENT SPECIFICATION

(11) **EP 1 998 689 B1**
(45) Date of publication and mention of the grant of the patent: **22.03.2017**
(21) Application number: 07757253.5
(22) Date of filing: 21.02.2007
(51) Int. Cl.: A61B 17/06

(54) **SUTURE PACKAGE**
VERPACKUNG FÜR NÄHMATERIAL
EMBALLAGE POUR FILS DE SUTURE

(30) Priority: 30.03.2006 US 393503
(43) Date of publication of application: 10.12.2008
(73) Proprietor: Ethicon, Inc, Somerville, NJ 08876-0151 (US)
(72) Inventor: CERWIN, Robert, J., Pipersville, Pennsylvania 18947 (US); DEY, Clifford, A., Reiglesville, Pennsylvania 18077 (US)
(74) Representative: Kirsch, Susan Edith
(86) International application number: PCT/US2007/062473
(87) International publication number: WO 2007/117769

(56) References cited:
- US-A- 6 047 815
- US-A- 6 135 272

## Description

### BACKGROUND OF THE INVENTION

### 1. Field of the Invention

The present invention relates to packaging, and more particularly, to packages used for surgical sutures.

### 2. Background Discussion

Packaging for surgical sutures and needles is well known in the art. Conventional packages serve several useful functions, including protecting the needles and sutures during handling, shipping, and storage, and also to facilitate dispensing of the sutures during surgery or other medical procedures. Known packages may be used for surgical sutures armed with surgical needles, or for unarmed surgical sutures without needles.

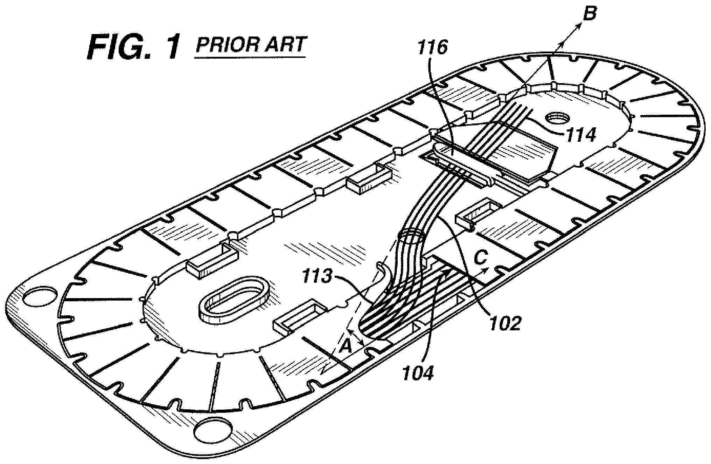

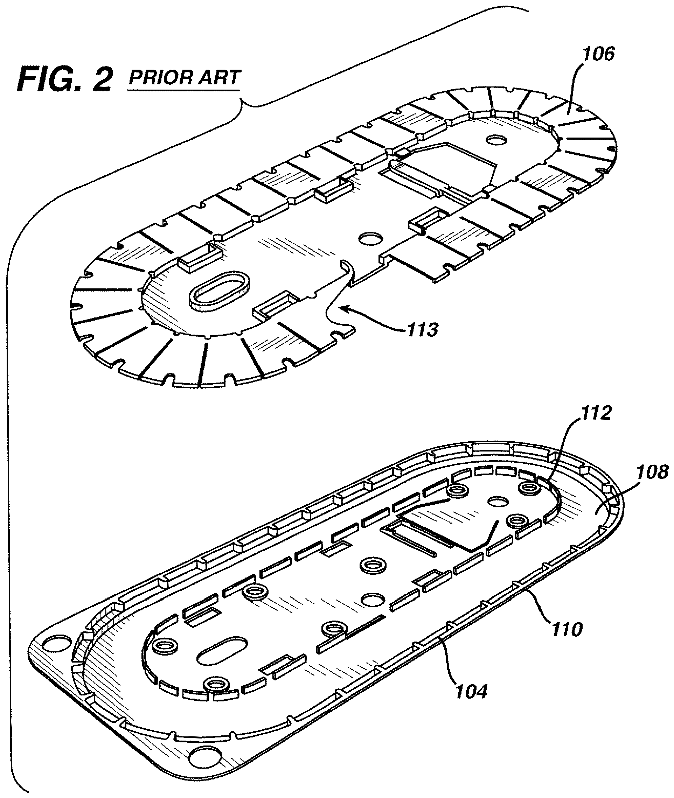

One known type of suture package is a tray package having a winding channel. These packages are typically oval in shape with inner and outer walls forming an oval winding channel. Such packages are typically molded from plastic, and are mounted onto a winding fixture with the sutures subsequently wound into the winding channel. One prior art suture package of this type is illustrated in Figs. 1 and 2, and is described in detail in U.S. Patent No. 6,047,815. Referring to Fig. 1, the package includes multiple suture strands 102 wound and positioned within winding channel 104. As better shown in Fig. 2, the package has a top portion 106 and a bottom portion 108 having an outer wall 110 and inner wall 112 that, when joined together, form the winding channel 104. The sutures exit the winding channel through a dispensing port 113 that is an opening in the top portion 106. The sutures exit the dispensing port and extend to their respective ends 114 where they are held in place by a holding means such as a clip 116 or the like. In these known packages, sutures are dispensed (by pulling on the free ends 114) at an angle A of less than 90 degrees from the path of the suture upon exiting the dispensing port. In other words, the
dispensing direction of the sutures ("dispensing direction" as indicated by arrow B) is at a substantially similar angle (as illustrated substantially less than 90 degrees) to the direction at which the sutures exit the dispensing port ("dispensing port direction" as illustrated by arrow C).

US 6,135,272 discloses a package for sutures having a winding channel. The package has a base member and a cover member that is mounted to the base member. The winding channels are supposed to allow sutures to be packaged using high-speed packaging processes. The winding channels are also supposed to overcome problems associated with the removal of sutures.

With multi-strand suture packages of the type described above, it is important that one be able to readily remove individual suture strands one at a time. With the above-described suture package, however, a typical problem is that pulling on one suture strand causes one or more other strands to follow. In other words, when one suture strand is pulled, others tend to be dragged, or at least partially dragged out with the one suture.

Accordingly, there is a need in the art for a new and improved suture package that better allows a single suture strand to be removed without affecting remaining suture strands in the package.

### SUMMARY OF THE INVENTION

The present invention provides a suture package including a cover member having a top side and a bottom side, a cantilever arm on the top side thereof, and a dispensing port opening therethrough; and a base member having a top side, a bottom side, an outer peripheral wall extending upwardly from the top side about a first peripheral location, and an inner peripheral wall extending upwardly from the top side about a second peripheral location that is inward of the first peripheral location. The base member is coupled with the cover member such that a portion of the bottom surface of the cover member, in conjunction with a portion of the top surface and inner and outer peripheral walls of the base member form a continuous channel therebetween, and such that the dispensing port opening of the cover member is aligned with and provides access into the continuous channel. The package further includes a plurality of sutures extending substantially along the length of and within the channel. An end region of each suture extends out of the channel through the dispensing port openingto a distal end that is removably secured to the package by the cantilever arm. A pathway followed by each suture from the point at which the suture extends out from the suture channel through the dispensing port opening to the point at which the suture is removably secured to the package by the cantilever arm, changes direction by at least 90 degrees.

The channel may be substantially oval or circular in shape, and the inner and/or outer peripheral walls may further consist of a plurality of consecutive wall members having spaces therebetween.

In one embodiment, the pathway changes direction by at least approximately 180 degrees.

In yet another embodiment the cantilever arm of the cover member is positioned inward of the inner peripheral wall. The suture package may further include first and second ends, and first and second side edges, wherein the suture distal ends are held by the holding device such that they can be withdrawn by a user in a direction substantially parallel to the first and second side edges.

In yet another embodiment, the dispensing port is positioned substantially along the first lateral edge of the suture package and the sutures exit the dispensing port in a direction toward the second end of the suture package. The cantilever arm may further be positioned closer to the first end of the suture package than the dispensing port.

Also provided is a suture package having a cover member and a base member positioned adjacent one another and configured so as to form a continuous channel therebetween, and a cantilever arm positioned on a top surface of the cover member. The continous channel has a substantially oval configuration, and the cover member has a dispensing port opening therethrough providing access into the channel. The package includes a plurality of sutures extending substantially along the length of and within the channel, with an end region of each suture extending out of the channel through the dispensing port opening to a distal end that is removably secured to the package by the holding device. Each suture exits through the dispensing port opening and changes direction by at least 90 degrees before being held in place by the holding device.

In one embodiment, the sutures change direction by at least approximately 180 degrees before being held in place by the cantilever arm, and in yet another embodiment the holding device is positioned inward of the channel.

In an alternate embodiment, the suture package further comprises first and second ends, and first and second side edges, wherein the suture distal ends are held by the cantilever arm such that they can be withdrawn by a user in a direction substantially parallel to the first and second side edges. The dispensing port may further be positioned substantially along the first lateral edge of the suture package and the sutures exit the dispensing port in a direction toward the second end of the suture package. In yet another embodiment, the cantilever arm is positioned closer to the first end of the suture package than the dispensing port.

### BRIEF DESCRIPTION OF THE DRAWINGS

**Figure 1** **is perspective view of a prior art suture package having surgical sutures therein;**
Figure 2 is an exploded perspective view of the prior art suture package of Fig. 1;
Figure 3 is a perspective view of a suture package according to the present invention having surgical sutures therein; and
Figure 4 is an exploded perspective view of the suture package of Fig. 3.

### DETAILED DESCRIPTION OF THE INVENTION

Before explaining the present invention in detail, it should be noted that the invention is not limited in its application or use to the details of construction and arrangement of parts illustrated in the accompanying drawings and description. The invention as illustrated may be implemented or incorporated in other embodiments, variations and modifications, and may be practiced or carried out in various ways.

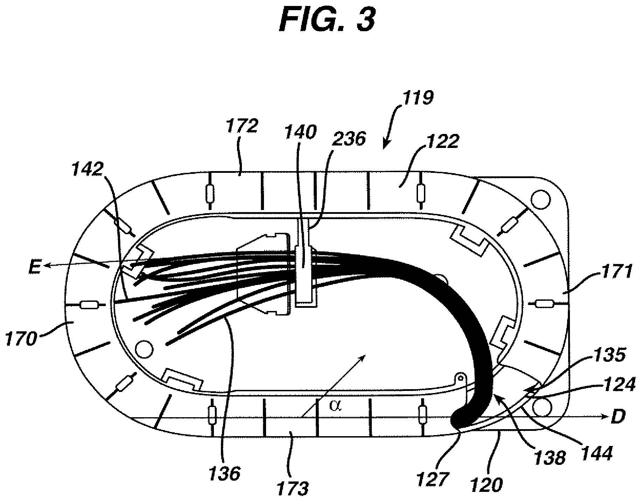

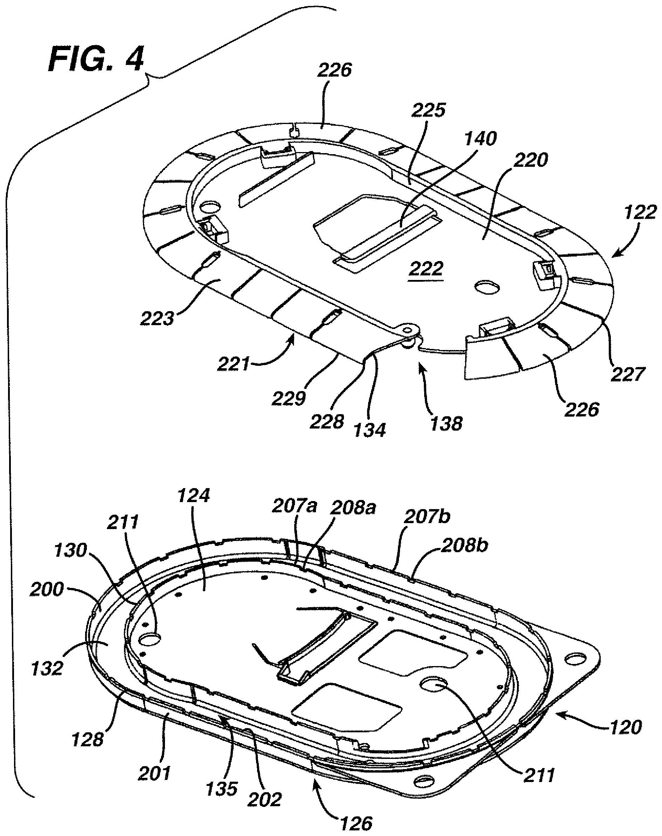

Referring now to Figs. 3 and 4, one embodiment of a suture package 119 according to the present invention has a first end 170, a second end 171, and first and second side edges 172, 173, and is preferably substantially oval in overall configuration, although other configurations (circular, polygonal, rectangular with rounded corners etc.) may be suitable as well. The suture package further includes a base member or portion 120 and a cover member 122. The base member 120 has a top side 124 and a bottom side 126, an outer periphery 127. Projecting upwardly from the top surface of the base member is an outer peripheral wall 128 and an inner peripheral wall 130. The outer peripheral wall and inner peripheral wall, in conjunction with the winding channel lower surface 132 of the base member and winding channel upper surface (defined by a portion 134 of the lower surface or bottom side 221 of channel cover member 122) define an enclosure or winding channel 135 within which the suture strands 136 are placed. In the illustrated embodiment, the winding channel is substantially oval in shape. The winding channel upper surface of the channel cover member has an opening therein 138, through which the wound suture strands 136 exit the winding channel as shown in Fig. 3.

The upwardly extending outer peripheral member 128 has an inner side 200, outer side 201 and top side 202. The inner and outer peripheral walls 130, 128 may consist of a plurality of members 207a, 207b that extend upwardly from the base member 120, and that may be separated or partially separated by openings or partial openings 208a, 208b. Extending through the base member 120 interior of the interior peripheral wall are one or more winding pin locating holes 211 for locating the base member on the winding head.

The cover member 122 also has a top side 220, a bottom side 221, and includes a first portion 222 that is centrally located and a suture channel cover portion 223 that extends about the peripheral area of the cover member 122. The inner edge 225 of the suture channel cover portion 223 preferably extends upwardly from the top surface of the first portion 222 as illustrated. The suture channel cover portion may further be comprised of multiple cantilevered cover members 226 separated from each other by a space 227. Further, a rim 228 may project downwardly from the outermost peripheral edge 229 of the cover members 226.

The suture channel cover portion 223 is discontinuous around the periphery of the cover member in that there is an opening 138 forming a dispensing port through the suture channel cover portion. Mounted within the first portion 222 of the cover member 122 is a cantilevered clip arm 140.

In contrast to prior art suture packages as shown in Figs. 1 and 2, the cantilevered clip arm 140 of the package shown in Figs. 3 and 4 is located in a position such that suture strands in the suture package that exit the suture channel through opening 138 must extend in an arc approximating a 180 degree turn before their ends are held in place by clip arm 140, as is shown in Fig. 3. In other words, the direction at which the suture strands 136 exit the opening or dispensing port 138 is shown generally by arrow D, whereas the direction at which they exit the holding element or clip arm 140 shown generally by arrow E. As is readily apparent in the illustrated embodiment, the suture will travel around an angle α of approximately 180 degrees between the point and direction at which they exit the dispensing port and the point and direction at which they can be removed by the user. Although approximately 180 degrees is the preferred arc, arcs of at least approximately 90 degrees may be suitable as well.

By forcing the suture strand to travel along a pathway representing at least a 90 degree, and preferably approximately 180 degree, bend before being removed entirely from the package, the "dragging" problem associated with prior art packages has been significantly reduced.

It will be apparent from the foregoing that, while particular forms of the invention have been illustrated and described, various modifications can be made without departing from the scope of the invention. Accordingly, it is not intended that the invention be limited, except as by the appended claims.

## Claims

1. A suture package (119) comprising:
a cover member (122) having a top side (220) and a bottom side (221), a holding device on the top side thereof, and a dispensing port (138) opening therethrough;
a base member (120) having a top side (124), a bottom side (126), an outer peripheral wall (128) extending upwardly from the top side (124) about a first peripheral location, and an inner peripheral wall (130) extending upwardly from the top side (124) about a second peripheral location that is inward of the first peripheral location, the base member (120) being coupled with the cover member (122) such that a portion of the bottom surface (126) of the cover member (122), in conjunction with a portion of the top surface (124) and inner (130) and outer (128) peripheral walls of the base member (120) form a continuous channel (135) therebetween, and such that the dispensing port (138) opening of the cover member (122) is aligned with and provides access into the continuous channel (135); and
a plurality of sutures extending substantially along the length of and within the channel (135), with an end region of each suture extending out of the channel through the dispensing port (138) opening to a distal end that is removably secured to the package by the holding device, **characterized in that**:
the holding device is a cantilever arm (140), and
wherein a pathway followed by each suture from the point at which the suture extends out from the channel (135) through the dispensing port (138) opening to the point at which the suture is removably secured to the package by the cantilever arm (140), changes direction by at least 90 degrees, such that the cantilever arm is configured to force the suture to travel along the pathway representing at least a 90 degree bend when the suture is removed from the package.

2. The package according to claim 1, wherein the channel is substantially oval or circular in shape.

3. The package according to claim 2, wherein the inner and/or outer peripheral walls (128 / 130) are comprised of a plurality of consecutive wall members (207a / 207b) having spaces therebetween.

4. The package according to claim 1, wherein the pathway changes direction by at least approximately 180 degrees.

5. The package according to claim 1, wherein the cantilever arm (140) of the cover member (122) is positioned inward of the inner peripheral wall (130).

6. The package according to claim 5, wherein the suture package further comprises first (170) and second (171) ends, and first (173) and second (172) side edges, and wherein the suture distal ends are held by the cantilever arm (140) such that they can be withdrawn by a user in a direction substantially parallel to the first and second side edges (172 / 173).

7. The package according to claim 6, wherein the dispensing port (138) is positioned substantially along the first lateral edge (173) of the suture package and the sutures exit the dispensing port (138) in a direction toward the second end (171) of the suture package.

8. The package according to claim 7, wherein the cantilever arm (140) is positioned closer to the first end (170) of the suture package than the dispensing port (138).

9. A suture package comprising:
a cover member (122) and a base member (120) positioned adjacent one another and configured so as to form a continuous channel (135) therebetween, and a holding device positioned on a top surface (220) of the cover member (122), the continuous channel (135) having a substantially oval configuration, and the cover member (122) having a dispensing port (138) opening therethrough providing access into the channel (135);
a plurality of sutures extending substantially along the length of and within the channel (135), with an end region of each suture extending out of the channel (135) through the dispensing port (138) opening to a distal end that is removably secured to the package by the holding device, **characterized in that**:
the holding device is a cantilever arm (140), and
wherein each suture exits through the dispensing port (138) opening and changes direction by at least 90 degrees before being held in place by the cantilever arm (138), such that the cantilever arm is configured to force the suture to travel along a pathway representing at least a 90 degree bend when the suture is removed from the package.

10. The package according to claim 1, wherein the sutures change direction by at least approximately 180 degrees before being held in place by the cantilever arm (140).

11. The package according to claim 9, wherein the cantilever arm (140) is positioned inward of the channel (135).

12. The package according to claim 11, wherein the suture package further comprises first and second ends (170 / 171), and first and second side edges (172 / 173), and wherein the suture distal ends are held by the cantilever arm (140) such that they can be withdrawn by a user in a direction substantially parallel to the first and second side edges (172 / 173).

13. The package according to claim 12, wherein the dispensing port (138) is positioned substantially along the first lateral edge (173) of the suture package and the sutures exit the dispensing port (138) in a direction toward the second end (171) of the suture package.

14. The package according to claim 13, wherein the cantilever arm (140) is positioned closer to the first end (170) of the suture package than the dispensing port (138).

## Patentansprüche

1. Nahtmaterialverpackung (119), umfassend:
ein Abdeckglied (122) mit einer oberen Seite (220) und einer unteren Seite (221), einer Haltevorrichtung auf der oberen Seite davon und einer Ausgabelochöffnung (138) dort hindurch,
ein Basisglied (120) mit einer oberen Seite (124), einer unteren Seite (126), einer äußeren Umfangswand (128), die sich von der oberen Seite (124) um einen ersten Umfangsort nach oben erstreckt, und einer inneren Umfangswand (130), die sich von der oberen Seite (124) um einen zweiten Umfangsort, der einwärts vom ersten Umfangsort liegt, nach oben erstreckt, wobei das Basisglied (120) so mit dem Abdeckglied (122) gekoppelt ist, dass ein Abschnitt der unteren Fläche (126) des Abdeckglieds (122) gemeinsam mit einem Abschnitt der oberen Fläche (124) und der inneren (130) und der äußeren (128) Umfangswand des Basisglieds (120) dazwischen einen durchgehenden Kanal (135) bildet, und so, dass die Ausgabelochöffnung (138) des Abdeckglieds (122) auf den durchgehenden Kanal (135) ausgerichtet ist und Zugang in diesen bereitstellt, und
eine Vielzahl von Nahtmaterialfäden, die sich im Wesentlichen entlang der Länge des Kanals (135) und in diesem erstrecken, wobei sich ein Endbereich jedes Nahtmaterialfadens aus dem Kanal durch die Ausgabelochöffnung (138) zu einem distalen Ende erstreckt, das durch die Haltevorrichtung entfernbar an der Verpackung befestigt ist, **dadurch gekennzeichnet, dass**
die Haltevorrichtung ein Kragarm (140) ist, und
wobei eine Bahn, der jeder Nahtmaterialfaden von der Stelle, an der sich der Nahtmaterialfaden aus dem Kanal (135) erstreckt, durch die Ausgabelochöffnung (138) zu der Stelle, an der der Nahtmaterialfaden durch den Kragarm (140) entfernbar an der Verpackung befestigt ist, folgt, um mindestens 90 Grad die Richtung wechselt, so dass der Kragarm dazu konfiguriert ist, den Nahtmaterialfaden dazu zu zwingen, sich entlang der mindestens eine 90-Grad-Biegung darstellenden Bahn zu bewegen, wenn der Nahtmaterialfaden aus der Verpackung entfernt wird.

2. Verpackung nach Anspruch 1, wobei der Kanal im Wesentlichen oval oder kreisförmig ist.

3. Verpackung nach Anspruch 2, wobei die innere und/oder die äußere Umfangswand (128/130) aus einer Vielzahl von aufeinanderfolgenden Wandgliedern (207a/207b) mit dazwischenliegenden Räumen bestehen.

4. Verpackung nach Anspruch 1, wobei die Bahn um mindestens ungefähr 180 Grad die Richtung wechselt.

5. Verpackung nach Anspruch 1, wobei der Kragarm (140) des Abdeckglieds (122) von der inneren Umfangswand (130) nach innen positioniert ist.

6. Verpackung nach Anspruch 5, wobei die Nahtmaterialverpackung ferner ein erstes (170) und ein zweites (171) Ende und einen ersten (173) und einen zweiten (172) Seitenrand umfasst und wobei die distalen Nahtmaterialfadenenden so durch den Kragarm (140) gehalten werden, dass sie von einem Benutzer in einer im Wesentlichen parallel zu dem ersten und zweiten Seitenrand (172/173) verlaufenden Richtung herausgezogen werden können.

7. Verpackung nach Anspruch 6, wobei das Ausgabeloch (138) im Wesentlichen entlang dem ersten Seitenrand (173) der Nahtmaterialverpackung positioniert ist und die Nahtmaterialfäden in einer zum zweiten Ende (171) der Nahtmaterialverpackung verlaufenden Richtung aus dem Ausgabeloch (138) austreten.

8. Verpackung nach Anspruch 7, wobei der Kragarm (140) näher am ersten Ende (170) der Nahtmaterialverpackung positioniert ist als das Ausgabeloch (138).

9. Nahtmaterialverpackung, umfassend:
ein Abdeckglied (122) und ein Basisglied (120), die einander benachbart positioniert und so konfiguriert sind, dass sie zwischen sich einen durchgehenden Kanal (135) bilden, und eine Haltevorrichtung, die auf einer oberen Fläche (220) des Abdeckglieds (122) positioniert ist, wobei der durchgehende Kanal (135) eine im Wesentlichen ovale Konfiguration hat und das Abdeckglied (122) eine Ausgabelochöffnung (138) dort hindurch hat, die Zugang in den Kanal (135) bereitstellt,
eine Vielzahl von Nahtmaterialfäden, die sich im Wesentlichen entlang der Länge des Kanals (135) und in diesem erstrecken, wobei sich ein Endbereich jedes Nahtmaterialfadens aus dem Kanal (135) durch die Ausgabelochöffnung (138) zu einem distalen Ende erstreckt, das durch die Haltevorrichtung entfernbar an der Verpackung befestigt ist, **dadurch gekennzeichnet, dass**
die Haltevorrichtung ein Kragarm (140) ist, und
wobei jeder Nahtmaterialfaden durch die Ausgabelochöffnung (138) austritt und um mindestens 90 Grad die Richtung wechselt, bevor er durch den Kragarm (138) festgehalten wird, so dass der Kragarm dazu konfiguriert ist, den Nahtmaterialfaden dazu zu zwingen, sich entlang einer mindestens eine 90-Grad-Biegung darstellenden Bahn zu bewegen, wenn der Nahtmaterialfaden aus der Verpackung entfernt wird.

10. Verpackung nach Anspruch 1, wobei die Nahtmaterialfäden um mindestens ungefähr 180 Grad die Richtung wechseln, bevor sie durch den Kragarm (140) festgehalten werden.

11. Verpackung nach Anspruch 9, wobei der Kragarm (140) vom Kanal (135) nach innen positioniert ist.

12. Verpackung nach Anspruch 11, wobei die Nahtmaterialverpackung ferner ein erstes und ein zweites (170/171) Ende und einen ersten und einen zweiten (172/173) Seitenrand umfasst und wobei die distalen Nahtmaterialfadenenden so durch den Kragarm (140) gehalten werden, dass sie von einem Benutzer in einer im Wesentlichen parallel zu dem ersten und zweiten Seitenrand (172/173) verlaufenden Richtung herausgezogen werden können.

13. Verpackung nach Anspruch 12, wobei das Ausgabeloch (138) im Wesentlichen entlang dem ersten Seitenrand (173) der Nahtmaterialverpackung positioniert ist und die Nahtmaterialfäden in einer zum zweiten Ende (171) der Nahtmaterialverpackung verlaufenden Richtung aus dem Ausgabeloch (138) austreten.

14. Verpackung nach Anspruch 13, wobei der Kragarm (140) näher am ersten Ende (170) der Nahtmaterialverpackung positioniert ist als das Ausgabeloch (138).

## Revendications

1. Emballage (119) pour fils de suture, comprenant :
un élément de couvercle (122) comportant un côté de dessus (220) et un côté de dessous (221), un dispositif de fixation sur le côté supérieur de celui-là et un orifice de distribution (138) s'ouvrant à travers celui-là ;
un élément de base (120) comportant un côté de dessus (124), un côté de dessous (126), une paroi périphérique extérieure (128) s'étendant vers le haut depuis le côté de dessus (124) autour d'un premier emplacement périphérique et une paroi périphérique intérieure (130) s'étendant vers le haut depuis le côté de dessus (124) autour d'un second emplacement périphérique qui est plus à l'intérieur que le premier emplacement périphérique, l'élément de base (120) étant accouplé avec l'élément de couvercle (122) de telle sorte qu'une partie de la surface inférieure (126) de l'élément de couvercle (122), conjointement avec une partie de la surface supérieure (124) et des parois périphériques intérieure (130) et extérieure (128) de l'élément de base (120), constitue entre elles un canal (135) continu, et de telle sorte que l'ouverture de l'orifice de distribution (138) de l'élément de couvercle (122) est aligné avec le canal (135) continu et assure un accès à celui-ci ; et
une pluralité de fils de suture s'étendant à l'intérieur du canal (135) sur pratiquement toute sa longueur, avec une zone terminale de chaque fil de suture s'étendant à l'extérieur du canal par l'ouverture de l'orifice de distribution (138) jusqu'à une extrémité distale qui est solidement fixée amovible à l'emballage par le dispositif de fixation,
**caractérisé en ce que** le dispositif de fixation est un bras en porte-à-faux (140), et
dans lequel le chemin suivi par chaque fil de suture, du point où le fil de suture sort du canal (135) par l'ouverture de l'orifice de distribution (138) au point où le fil de suture est solidement fixé amovible à l'emballage par le bras en porte-à-faux (140), change de direction d'au moins 90 degrés, de telle sorte que le bras en porte-à-faux est configuré pour forcer le fil de suture à se déplacer le long du chemin représentant un coude d'au moins 90 degrés quand on retire le fil de suture de l'emballage.

2. Emballage selon la revendication 1, dans lequel le canal est de forme sensiblement ovale ou circulaire.

3. Emballage selon la revendication 2, dans lequel les parois périphériques intérieure et/ou extérieure (128/130) sont constituées d'éléments (207a/207b) de paroi qui se suivent et comportent entre eux des espaces.

4. Emballage selon la revendication 1, dans lequel le chemin change de direction d'au moins 180 degrés environ.

5. Emballage selon la revendication 1, dans lequel le bras en porte-à-faux (140) de l'élément de couvercle (122) est placé vers l'intérieur de la paroi périphérique intérieure (130).

6. Emballage selon la revendication 5, dans lequel l'emballage pour fils de suture comprend en outre une première (170) et une seconde (171) extrémités et un premier (173) et un second (172) bords latéraux, et dans lequel les extrémités distales des fils de suture sont tenues par le bras en porte-à-faux (140) de telle sorte qu'un utilisateur peut les retirer dans une direction sensiblement parallèle aux premier et un second bords latéraux (172/173).

7. Emballage selon la revendication 6, dans lequel l'orifice de distribution (138) est placé pratiquement le long du premier bord latéral (173) de l'emballage pour fils de suture et les fils de suture sortent de l'orifice de distribution (138) dans la direction de la seconde extrémité (171) de l'emballage pour fils de suture.

8. Emballage selon la revendication 7, dans lequel le bras en porte-à-faux (140) est placé plus près de la première extrémité (170) de l'emballage pour fils de suture que l'orifice de distribution (138).

9. Emballage pour fils de suture comprenant :
un élément de couvercle (122) et un élément de base (120), juxtaposés l'un à l'autre et configurés de façon à constituer entre eux un canal (135) continu, et un dispositif de fixation placé sur la surface supérieure (220) de l'élément de couvercle (122), le canal (135) continu présentant une configuration sensiblement ovale, et l'élément de couvercle (122) comportant un orifice de distribution (138) qui le traverse et assure l'accès au canal (135) ;
une pluralité de fils de suture s'étendant à l'intérieur du canal (135) sur pratiquement toute sa longueur, avec une zone terminale de chaque fil de suture s'étendant à l'extérieur du canal (135) par l'ouverture de l'orifice de distribution (138) jusqu'à une extrémité distale qui est solidement fixée amovible à l'emballage par le dispositif de fixation,
**caractérisé en ce que** le dispositif de fixation est un bras en porte-à-faux (140), et
dans lequel chaque fil de suture sort par l'orifice de distribution (138) et change de direction d'au moins 90 degrés avant d'être maintenu en place par le bras en porte-à-faux (138), de telle sorte que le bras en porte-à-faux est configuré pour forcer le fil de suture à se déplacer le long d'un chemin représentant un coude d'au moins 90 degrés quand on retire le fil de suture de l'emballage.

10. Emballage selon la revendication 1, dans lequel les fils de suture changent de direction d'au moins 180 degrés environ avant d'être maintenus en place par le bras en porte-à-faux (140).

11. Emballage selon la revendication 9, dans lequel le bras en porte-à-faux (140) est placé vers l'intérieur du canal (135).

12. Emballage selon la revendication 11, dans lequel l'emballage de fils de suture comprend en outre des première et seconde extrémités (170/171) et des premier et second bords latéraux (172/173), et dans lequel les extrémités distales des fils de suture sont tenues par le bras en porte-à-faux (140) de telle sorte qu'un utilisateur peut les retirer dans une direction sensiblement parallèle aux premier et un second bords latéraux (172/173).

13. Emballage selon la revendication 12, dans lequel l'orifice de distribution (138) est placé pratiquement le long du premier bord latéral (173) de l'emballage pour fils de suture et les fils de suture sortent de l'orifice de distribution (138) dans la direction de la seconde extrémité (171) de l'emballage pour fils de suture.

14. Emballage selon la revendication 13, dans lequel le bras en porte-à-faux (140) est placé plus près de la première extrémité (170) de l'emballage pour fils de suture que l'orifice de distribution (138).
